# EUROPEAN PATENT SPECIFICATION

(11) **EP 1 771 213 B1**
(45) Date of publication and mention of the grant of the patent: **28.04.2010**
(21) Application number: 05761121.2
(22) Date of filing: 14.07.2005
(51) Int. Cl.: A61L 17/10, A61L 17/04

(54) **ELONGATED SURGICAL REPAIR PRODUCT BASED ON UHMWPE FILAMENTS**
LÄNGLICHES CHIRURGISCHES REPARATURPRODUKT AUF BASIS VON UHMWPE FILAMENTEN
DISPOSITIF ALLONGÉ DE RÉPARATION À BASE DES FILAMENTS EN PE UHMW

(30) Priority: 27.07.2004 EP 04077163; 27.07.2004 US 591098 P
(43) Date of publication of application: 11.04.2007
(73) Proprietor: DSM IP Assets B.V., 6411 TE Heerlen (NL)
(72) Inventor: SMIT, Leo, NL-6438 AE Oirsbeek (NL)
(74) Representative: Dorrestijn, Antoon
(86) International application number: PCT/EP2005/007817
(87) International publication number: WO 2006/010522

(56) References cited:
- EP-A- 0 213 208
- EP-A- 0 561 108
- EP-A- 1 293 218
- GB-A- 2 042 414
- US-A- 5 106 563
- US-A- 5 540 703

## Description

The invention relates to an elongated surgical repair product comprising an elongated member of core-sheath structure, the core containing a plurality of high-strength ultra-high molar mass polyethylene (UHMWPE) filaments.

The invention also relates to a process of making such an elongated surgical repair product.

Such a product is known from EP 1293218 A1. This publication discloses a suture strand for use as a surgical suture or ligament, which suture strand comprises a core of twisted strands of ultra-high molar mass polyethylene (UHMWPE) filaments, surrounded by a multifilament braided sheath made of UHMWPE filaments and filaments of one or more long chain synthetic polymers, preferably polyester. The suture typically contains 60-67 mass% of UHMWPE filaments. The suture can optionally be coated with various materials to improve handling properties.

In EP 0561108 A2 surgical repair products are disclosed, like braided, woven or knitted tapes, or hollow or spiroid braids, which are constructed in whole or in part from UHMWPE filaments. The products may be applied as sutures for repairing soft body tissue, but also as tapes or bands to retain bone portions together. More specifically, a suture is described, which comprises a flexible elongated member containing a core of untwisted UHMWPE filaments surrounded by a braided sheath made from air-entangled polyester yarn.

An elongated surgical repair product is understood to be an article for use as for example a surgical suture for repairing soft body tissue, or as a cable, tape, ribbon or band for repairing or retaining body parts like bones; and the elongated product is of length dimension much larger than its cross-sectional dimensions (width, thickness). The repair product comprises at least one elongated member, which is generally the load-bearing component and made from high-strength filaments; and may further comprise for example an anchor or a needle, a coating material, etc. High-strength filaments are defined as being filaments of tensile strength of more than 1.0 GPa.

Elongated surgical repair products like sutures have been made over time from a variety of materials, including flax, hair, cotton, silk, animal gut, and synthetic materials like polyesters, polyamides, and polyolefins like polyethylene or polypropylene. The material used may be absorbable or non-absorbable. Non-absorbable products are not dissolved or degraded by the body's natural action after implantation. Relevant material properties for use in sutures and other repair products include tensile strength, flexibility, elasticity, wettability, and other surface properties. A relatively new material for making non-absorbable surgical repair products, is multifilament yarn made from ultra-high molar mass polyethylene (UHMWPE). The main advantages of this material include its biocompatibility, its relatively low density, and especially its very high tensile strength; or better its high strength on mass basis (tenacity). For use in sutures the knotting behaviour of this material has been indicated to be less favourable; i.e. it would not provide a desired combination of knot tie down and knot stability or knot slip.

The core-sheath suture containing UHMWPE and polyester filaments known from EP 1293218 A1 is indicated to provide improved knot tie down properties. The presence of a braided sheath made from less strong fibres, however, reduces the strength efficiency of the UHMWPE filaments.

There is an ongoing need in industry for an elongated surgical repair product that effectively utilizes the high strength (tenacity) of UHMWPE filaments and shows improved knotting characteristics.

The object of the present invention is therefore to provide an elongated surgical repair product comprising an elongated member based on high-strength UHMWPE filaments that has high tensile strength, good flexibility and favourable knotting characteristics.

This object is achieved according to the invention with a surgical repair product wherein the elongated member has a UHMWPE sheath that is non-porous.

The elongated surgical repair product according to the invention shows high flexibility, improved knot strength and knot slip and higher tensile strength on a mass basis. Another advantage of the surgical repair product according to the invention is that its surface is smoother than that of a product containing a member with a braided sheath, resulting in less damaging of tissue during or after implantation. A further related advantage of the non-porous sheath is that it is less prone to harbouring of infectious matter, e.g. of bacteria breeding in small voids. The member also shows little fraying and improved resistance to ravelling.

From WO 86/00020 A1 a surgical suture comprising a core of UHMWPE filaments and a sheath is known, which sheath is a coating layer of a film-forming conventional surgical suture material; but a UHMWPE sheath is not disclosed nor suggested in this publication. In addition, such conventional sheath layer does not contribute to the strength of the suture.

The surgical repair product according to the invention comprises an elongated member containing UHMWPE filaments, and a UHMWPE sheath. The UHMWPE in core and sheath need not be the same polymer grade, but in a preferred embodiment they are of substantially the same composition and molar mass. Within the context of the present application ultra-high molar mass polyethylene (UHMWPE) is understood to be polyethylene with an intrinsic viscosity (IV, as determined according to method PTC-179 (Hercules Inc. Rev. Apr. 29, 1982) at 135°C in decalin, with dissolution time of 16 hours, with anti-oxidant DBPC in an amount of 2 g/I solution, and the viscosity at different concentrations extrapolated to zero concentration) of above 5 dl/g. Particularly suitable, especially for the filaments, is UHMWPE with IV of between about 8 and 40 dl/g, more preferably between 10 and 30, or 12 and 28, or between 15 and 25 dl/g. These ranges represent an optimum in polymer processability and filaments properties. Intrinsic viscosity is a measure for molar mass (also called molecular weight) that can more easily be determined than actual molar mass parameters like Mn and Mw. There are several empirical relations between IV and Mw, but such relation is highly dependent on molar mass distribution. Based on the equation Mw = 5.37 x 10⁴ [IV]^{1.37} (see EP 0504954 A1) an IV of 8 dl/g would be equivalent to Mw of about 930 kg/mol.

Preferably, the UHMWPE is a linear polyethylene with less than one branch or side chain per 100 carbon atoms, and preferably less than one side chain per 300 carbon atoms, a branch usually containing at least 10 carbon atoms. The linear polyethylene may further contain up to 5 mol% of one or more comonomers, such as alkenes like propylene, butene, pentene, 4-methylpentene or octene.

In a preferred embodiment, the UHMWPE contains a small amount of relatively small groups as side chains, preferably a C1-C4 alkyl group. It is found that a filament from UHMWPE with a certain amount of such groups show reduced creep behaviour. Too large a side chain, or too high an amount of side chains, however, negatively affects the processing and especially the drawing behaviour of the filaments. For this reason, the UHMWPE preferably contains methyl or ethyl side chains, more preferably methyl side chains. The amount of side chains is preferably at least 0.3, 0.5, more preferably at least 1 per 1000 carbon atoms, and preferably at most 20, more preferably at most 10 per 1000 carbon atoms.

The UHMWPE can be a single polymer grade, but also a mixture of two or more different grades, e.g. differing in IV or molar mass distribution, and/or number of side chains.

The UHMWPE polymer may further contain usual amounts, generally less than 5 mass% of customary additives, such as anti-oxidants, thermal stabilizers, colorants, nucleating agents, flow promoters, catalyst residues etc.; as long as these components are suitable for the use in a surgical product. The filament (or sheath) may also contain other polymers, preferably polyolefinic polymers, like other polyethylenes, polypropylenes, or their copolymers, including rubbery copolymers like EPDM, EPR, etc. The amount of such other polymer is always lower than the amount of UHMWPE in the filament or sheath, and is preferably not more than 30% of the UHMWPE.

The core of the elongated member contains a plurality of high-strength UHMWPE filaments. Filaments are herein understood to be of indefinite length; or at least to have about the same length as the elongated member. Preferably, the tensile strength of the filaments is more than 1.5, 2.0 or even more than 3.0 GPa. Tensile strength, also simply strength, is determined on multifilament yarns as specified in ASTM D885M, using a nominal gauge length of the fibre of 500 mm, a crosshead speed of 50%/min and Instron 2714 clamps, type Fibre Grip D5618C. Higher strength of the filaments also results in higher strength of the surgical product.

The UHMWPE filaments in the member in the product according to the invention may have a filament linear density or titre varying between wide ranges. A suitable titre for the filaments is between about 0.2 and 20 dtex per filament, preferably about 0.3 - 10 dtex, or 0.4 - 5 dtex; since this results in a favourable combination of improved strength and high flexibility of the member and product.

Suitable UHMWPE filaments can be made by a process generally referred to as gel spinning. Gel spinning of UHMWPE is well known to the person skilled in the art; and described in numerous publications, including EP 0205960 A, EP 0213208 A1, US 4413110, GB 2042414 A, EP 0200547 B1, EP 0472114 B1, WO 01/73173 A1, and Advanced Fiber Spinning Technology, Ed. T. Nakajima, Woodhead Publ. Ltd (1994), ISBN 1-855-73182-7, and references cited therein. Gel spinning is understood to include at least the steps of spinning at least one filament from a solution of ultra-high molecular weight polyethylene in a spin solvent; cooling the filament obtained to form a gel filament; removing at least partly the spin solvent from the gel filament; and drawing the filament in at least one drawing step before, during or after removing spin solvent. Suitable spin solvents include for example paraffins, mineral oil, kerosene or decalin. Spin solvent can be removed by evaporation, extraction, or by a combination of evaporation and extraction routes. The UHMWPE filaments preferably contain less than 800 ppm of residual amounts of spin solvent, more preferably less than 500, 250, or even less than 100 ppm.

The filaments in the core show no or only little adhesion to each other; that is most of the filaments can move or shift relative to each other, or be separated from each other. This can for example be examined in a test wherein the sheath or removed (eg with a sharp knife), or after cutting the member open in the length direction. The advantage hereof is that the flexibility of the member remains favourable, also for members of larger diameter (or suture size) having a substantially non-porous sheath.

The core may in addition to UHMWPE filaments also contain other filaments, but preferably not more than 40 mass% of the total amount of filaments, more preferably not more than 30, 20, or 10 mass%. In view of optimum strength of the member, the core preferably only contains high-strength filaments; more preferably only UHMWPE filaments. In a most preferred embodiment the member, that is core and sheath, essentially consists of UHMWPE, with only minor amounts of other components like additives or coatings being present.

The UHMWPE sheath layer of the elongated member in the product according to the invention being non-porous is understood to mean that no or hardly any pores or voids are present, or at least can be seen on the surface of the member, e.g. with a microscope; such that micro-organisms like bacteria will not easily find a place to grow. The non-porous sheath thus may have pores or voids smaller than micro-organisms.

With a view to further improving the knotting behaviour of the surgical product according to the invention, the elongated member preferably has a cross-sectional width to thickness ratio of more than 1.0; meaning the elongated member has a flattened or oblong cross-section. The cross-section may have various forms, and can for example be oval, (virtually) rectangular, or of more irregular geometry.

Suitable oblong members have a cross-sectional width to thickness ratio of between about 1.5 and 25. Examples include elongated members that have a core that is made from a woven or (hollow) braided construction from UHMWPE filaments or multifilament yarns, of tape- or ribbon-like appearance. The core may, on the other hand, also contain twisted or untwisted UHMWPE yarns, the UHMWPE sheath layer then also serves to stabilize a flattened structure. The cross-sectional width to thickness ratio of a member can conveniently be determined using a microscope.

Preferably, the cross-sectional width to thickness ratio is at least 2, 3, 4 or 5 to result in reduced knot slip. Too high a width/thickness ratio may make handling and knotting more difficult, the ratio is thus preferably at most 20, 15, or at most 10. The skilled person can find an optimum cross-sectional width to thickness ratio for a member of certain size or dimension by some routine experiments.

In a preferred embodiment, the non-porous sheath layer is the result of a heat treatment applied to a fibrous precursor member, during which treatment the filaments on the outside of the member partially melted on their surface and adhered together to form the substantially non-porous layer. An advantage hereof includes that the sheath layer is still oriented and contributes to the strength of the member. A flattened cross-section can in such case, for example, be formed by guiding a multifilament yarn over a bar to spread the filaments, and by heating and subsequently cooling it.

The relative thickness of the substantially non-porous UHMWPE sheath of the elongated member in the product according to the invention may vary between wide limits. It has been found that if a sheath layer is too thick in relation to the core comprising UHMWPE filaments, the flexibility of the member becomes too low, but this will generally be dependent on the size or dimensions of the member; a thin member as such being more flexible and thus more forgiving for a relatively thick sheath layer. In order to display the desired effect, the sheath layer preferably has a certain minimum thickness. A suitable minimum thickness for the sheath is found to be on the order of about 20 micrometer, preferably it is at least 25 micron; but the sheath layer may be much thicker. The smaller sutures typically have a diameter in the range of 20 to 70 micrometer if considered to be virtually round (USP 10-0 to 6-0), whereas larger surgical sutures or orthopaedic cables may have diameters of up to 1 or 2 mm or even higher. Therefore, the sheath forms at least about 5 mass% of the member, preferably at least 10, 15, 20, 25, or 30 mass%. On the other hand, the sheath forms preferably at most 95 mass% of the member, more preferably at most 90, 80, 70, 60, or even at most 50 mass% for high flexibility. Although for a low diameter member, e.g. diameter (or thickness) below 150 micrometer, the non-porous sheath may constitute virtually 100% of the member, a higher relative content of UHMWPE filaments is advantageous for optimising strength and knot strength of the product.

The surgical repair product according to the invention shows high tensile strength or tenacity, and preferably has a tenacity of at least 10 cN/dtex, more preferably of at least 15, 20, 25, or even more than 28 or 30 cN/dtex .

In a preferred embodiment of the invention the surgical repair product is a surgical suture, more preferably a suture of USP (United States Pharmacopeia) sizes 1 to 10-0. Such sutures combine strength higher than known sutures with favourable knotting properties.

The invention further relates to surgical sutures comprising an elongated member having a substantially non-porous outer surface of UHMWPE and a tensile strength of at least 20 cN/dtex, more preferably of at least 25 or even at least 28 cN/dtex. In a preferred embodiment such suture is of relatively low diameter, for example of size USP 4-0 to 11-0, and is specifically suitable for use in microsurgery. A cross-section of such suture member may show a core-sheath structure as described above, but may also be a substantially non-porous member without a core; for example as a result of all filaments of a precursor being thermally fused.

In another embodiment the product is an orthopaedic cable, very suitable to be used for holding together for example bone parts.

The invention further specifically relates to the use of an elongated member as defined and described above in various embodiments, for making an elongated surgical repair product having an advantageous combination of a.o. high tensile strength, good knotting behaviour, and high knot strength retention.

The invention further relates to a method of making an elongated surgical repair product according to the invention comprising a step wherein an elongated precursor member containing a plurality of high-strength ultra-high molar mass polyethylene (UHMWPE) filaments, is subjected to a heat-treatment while keeping the filaments under tension to form a UHMWPE sheath that is substantially non-porous around a core of UHMWPE filaments.

In WO 86/00020 A1 a surgical suture comprising a core of UHMWPE filaments and a sheath is made by coating the core with a polymer solution or polymer melt, the polymer being a film-forming conventional surgical suture material. Making a sheath from UHMWPE is not disclosed or suggested in this publication.

The structure of a precursor member for applying in the process according to the invention is not particularly critical. Suitable constructions of UHMWPE filaments include twisted multifilament yarns, or braided, woven or knitted constructions, or a hybrid construction. Preferably a braided precursor is used, since this has more initial coherence is shows better fusing behaviour. Suitable braided precursors include circular or tubular braids, but also spiroid braid or flat braid constructions can be applied. In general, for making relatively thin repair products a simple precursor construction is preferred, whereas for thicker products more complicated constructions, like kern-mantle (twisted filaments in core - braided sheath), or braid-on-braid (also called double braided) cords, can be applied.

The conditions, like temperature, residence time and tension level in the process according to the invention are selected to be such that the filaments will soften or even start to melt predominantly at their outer surface, but without loosing their molecular orientation. This allows them to combine and adhere to each other at least at the surface of the precursor member, and to form into a sheath, which sheath is substantially non-porous. Conditions useful for the surface combining or fusing process include a temperature or series of oven temperatures within the melting point range of the filament UHMWPE polymer that allows for forming a sheath and core structure during the exposure period. The temperature at which the process is carried out is preferably within the range from about 150° C up to about 157° C for gel spun UHMWPE filaments exhibiting a relaxed melting range of 138° to about 162° C as measured by DSC at a 20° C/minute scan rate. Residence times during which the precursor is exposed to the oven temperature are for example within the range from about 6 seconds to about 150 seconds. In a special embodiment of the invention, the filaments are kept under tension by drawing them during heat exposure, preferably with a draw ratio of about 1.1 to 2.5. To prevent that the precursor is exposed to too much heat, the temperature profile applied can be adjusted, e.g. by applying a lower initial temperature, for example in the range 135-150 °C. The thickness of the sheath being formed can be controlled by increasing or decreasing the temperature, and/or by increasing or decreasing the residence time. The skilled man can find favourable settings by some routine experimentation. Precursor members of relatively low diameter may also be made into a member wherein substantially all filaments are melted together, without completely melting each filament. Typical embodiments and preferences for the precursor member and resulting member are analogous to the description above for the surgical repair product.

In a special embodiment of the process according to the invention a solvent for UHMWPE is additionally applied to the surface of the precursor before or during the heat treatment, to enhance the process of making a sheath and core structure. Such solvent may include mineral oil (e.g. heat transfer grade mineral oil with an average molecular weight of 250-700 g/mol), paraffin oil, vegetable oil (e.g., coconut oil), or any other solvent for polyethylene, such as decalin, or toluene. Contact between the precursor and the solvent can be performed under ambient conditions (e.g., 20°-25° C.) or under elevated temperature conditions (e.g. up to about 100-150° C. or higher). This solvent is thought to act as a plasticiser, and enhances the efficiency of the sheath making process; permitting the process for making the sheath and core member to be performed at lower temperatures. After forming the sheath structure, the member may be subjected to an additional treatment at lower temperatures, which treatment aims to result in substantially complete removal of residual processing additives (including solvent used for spinning) and the applied solvent, or at least to below concentrations allowed as maximum for a surgical application. The treatment can be a heat treatment, in case of a volatile component; or an extraction treatment. Preferably, the member obtained contains less than 800 ppm of solvent, more preferably less than 500, 250, or even less than 100 ppm.

The invention also relates to a method of producing an elongated surgical repair product, wherein the method further comprises a step of incorporating a biologically active compound, like a medical drug into the member. This step of incorporating a compound can be done by adding for example the medical drug to the spin solvent wherein the UHMWPE is dissolved during the production via gel spinning of a UHMWPE solution into filaments. Another way to incorporate a medical drug into the member is to add the medical drug to the solvent before the heat treatment step, but also other routes are possible.

## Claims

1. Elongated surgical repair product comprising an elongated member of core-sheath structure, the core containing a plurality of high-strength ultra-high molar mass polyethylene (UHMWPE) filaments, **characterized in that** the elongated member has a UHMWPE sheath that is non-porous.

2. Product according to claim 1, wherein the member has a cross-sectional width to thickness ratio of more than 1.0.

3. Product according to claim 2, wherein the width to thickness ratio is between about 1.5 and 25.

4. Product according to any one of claims 1-3, wherein the sheath layer has a thickness of at least 20 micrometer.

5. Product according to any one of claims 1-4, wherein the UHMWPE sheath forms from about 10 to 90 mass% of the member.

6. Product according to any one of claims 1-5, wherein the UHMWPE filaments have a tensile strength of more than 2.0 GPa.

7. Product according to any one of claims 1-6, wherein the filaments have a linear density of about 0.3 to about 10 dtex per filament.

8. Product according to any one of claims 1-7 being a surgical suture.

9. Product according to any one of claims 1-8 being an orthopaedic cable.

10. Process of making an elongated surgical repair product according to any one of claims 1-7, comprising a step of subjecting an elongated precursor member containing a plurality of UHMWPE filaments to a heat treatment while keeping the filaments under tension, to form a UHMWPE sheath layer that is non-porous around a core of UHMWPE filaments.

11. Process according to claim 10, wherein the filaments in the precursor are kept under tension by drawing with a draw ratio of about 1.1 to 2.5.

## Patentansprüche

1. Längliches Produkt für die chirurgische Reparation, umfassend ein längliches Glied mit Kern-Mantel-Struktur, wobei der Kern mehrere hochfeste Filamente aus Polyethylen ultrahoher Molmasse (UHMWPE) enthält, **dadurch gekennzeichnet, daß** das längliche Glied über einen nichtporösen Mantel aus UHMWPE verfügt.

2. Produkt nach Anspruch 1, bei dem das Glied über ein Verhältnis der Querschnittsbreite zur -dicke von mehr als 1,0 verfügt.

3. Produkt nach Anspruch 2, bei dem das Verhältnis der Breite zur Dicke zwischen etwa 1,5 und 25 liegt.

4. Produkt nach einem der Ansprüche 1-3, bei dem die Mantelschicht über eine Dicke von mindestens 20 Mikrometer verfügt.

5. Produkt nach einem der Ansprüche 1-4, bei dem der Mantel aus UHMWPE etwa 10 bis 90 Massen-% des Glieds ausmacht.

6. Produkt nach einem der Ansprüche 1-5, bei dem die Filamente aus UHMWPE über eine Zugfestigkeit von mehr als 2,0 GPa verfügen.

7. Produkt nach einem der Ansprüche 1-6, bei dem die Filamente über einen Einzeltiter von etwa 0,3 bis etwa 10 dtex verfügen.

8. Produkt nach einem der Ansprüche 1-7, bei dem es sich um ein chirurgisches Nahtmaterial handelt.

9. Produkt nach einem der Ansprüche 1-8, bei dem es sich um ein orthopädisches Kabel handelt.

10. Verfahren zur Herstellung eines länglichen Produkts für die chirurgische Reparation gemäß einem der Ansprüche 1-7, bei dem man ein längliches, mehrere Filamente aus UHMWPE enthaltendes Vorgängerglied unter Spannungsbeibehaltung der Filamente einer Wärmebehandlung unterwirft und so um einen Kern von Filamenten aus UHMWPE herum eine nichtporöse Mantelschicht aus UHMWPE ausbildet.

11. Verfahren nach Anspruch 10, bei dem man zur Spannungsbeibehaltung der Filamente im Vorgänger etwa 1 zu 1,1-fach bis 1 zu 2,5-fach verstreckt.

## Revendications

1. Dispositif allongé de réparation chirurgicale comprenant un élément allongé d'une structure composée d'une âme et d'une gaine, l'âme comprenant une pluralité de filaments hautement résistants à base de polyéthylène de masse moléculaire très élevée (PEhpm), **caractérisé en ce que** l'élément allongé comporte une gaine PEhpm qui est non poreuse.

2. Produit selon la revendication 1, dans lequel l'élément présente un rapport largeur de coupe transversale à épaisseur supérieur à 1,0.

3. Produit selon la revendication 2, dans lequel le rapport largeur à épaisseur se situe entre 1,5 et 25.

4. Produit selon l'une quelconque des revendications 1 à 3, dans lequel la couche de la gaine possède une épaisseur d'au moins 20 micromètres.

5. Produit selon l'une quelconque des revendications 1 à 4, dans lequel la gaine PEhpm forme d'environ 10 à 90 % en masse de l'élément.

6. Produit selon l'une quelconque des revendications 1 à 5, dans lequel les filaments PEhpm possèdent une résistance à la traction supérieure à 2,0 GPa.

7. Produit selon l'une quelconque des revendications 1 à 6, dans lequel les filaments possèdent une densité linéaire d'environ 0,3 à environ 10 dtex par filament.

8. Produit selon l'une quelconque des revendications 1 à 7 qui est une suture chirurgicale.

9. Produit selon l'une quelconque des revendications 1 à 8 qui est un câble orthopédique.

10. Procédé de fabrication d'un dispositif allongé de réparation chirurgicale selon l'une quelconque des revendications 1 à 7, comprenant une étape de soumission d'un élément précurseur allongé contenant une pluralité de filaments PEhpm à un traitement thermique tout en gardant les filaments sous tension, pour former une couche de gaine PEhpm qui est non poreuse autour d'une âme de filaments PEhpm.

11. Procédé selon la revendication 10, dans lequel les filaments dans le précurseur sont gardés sous tension en les étirant avec un rapport d'étirage d'environ 1,1 à 2,5.
